# EUROPEAN PATENT SPECIFICATION

(11) **EP 4 226 203 B1**
(45) Date of publication and mention of the grant of the patent: **01.10.2025**
(21) Application number: 21787449.4
(22) Date of filing: 06.10.2021
(51) Int. Cl.: G02B 21/34, C12M 1/32, G02B 23/26, C12M 1/34

(54) **SAMPLE HOLDER ASSEMBLY FOR OPTICAL MICROSCOPY**
PROBENHALTERANORDNUNG FÜR OPTISCHE MIKROSKOPIE
ENSEMBLE PORTE-ÉCHANTILLONS POUR MICROSCOPIE OPTIQUE

(30) Priority: 06.10.2020 EP 20200305
(43) Date of publication of application: 16.08.2023
(73) Proprietor: Charité - Universitätsmedizin Berlin, 10117 Berlin (DE)
(72) Inventor: TEN, Foo, Wei, 10115 Berlin (DE); CONRAD, Christian, 14165 Berlin (DE); EILS, Roland, 12555 Berlin (DE); YANG, Li-Ling, 12555 Berlin (DE)
(74) Representative: Schulz Junghans Patentanwälte PartGmbB
(86) International application number: PCT/EP2021/077628
(87) International publication number: WO 2022/074084

(56) References cited:
- DE-A1- 102018 200 923
- US-A- 1 002 910
- US-A- 6 048 723

## Description

The invention relates to a sample holder assembly for use in optical microscopy, as well as a method for imaging a sample with the assembling a sample holder assembly. In the art various sample holders for use in optical microscopy are known. Particularly in biological applications, when tissue or cells are imaged, it is an important feature of the sample holders to provide culturing and imaging capabilities, such that the sample does not need to be transferred form a culture dish to the imaging holder.

For this reason, there are culture dishes having a glass bottom with a thin high-grade optical microscopy slide integrated, such that the dish can be transferred directly to the microscope. However, such dishes are expensive and not particularly versatile in terms of different microscopy modes and therefore set hard limits to the possible imaging modalities.

From WO 2014/180884 A1 a microscope module for imaging a sample is known that comprises a sample holder that has a transparent bottom portion formed by a flexible membrane that protrudes further down toward the bottom side of the holder when a liquid is filled in the sample holder such that a sample volume by the protruding membrane. Any imaging performed on this sample holders has to specifically adapt the optics to the protruding curved membrane surface.

Moreover, as the membrane is glued to the walls of the sample holder, the sample holder is essentially designed for one-time use only.

US 1002910 A discloses a mounting device for objects comprising three parts; a first and a second member as well as pliable sheet. The first and the second member each have side walls that surround a volume, wherein the second member's side walls are slightly less in dimension than the first member's side walls so that the members may be fitted into each other with their side walls. The pliable sheet may be arranged between the side walls such that it is clamped between the side walls.

US 6048723 A teaches cell culture plate having a flexible membrane sandwiched between a base and a body. Each of the base and body have a plurality of mutually aligned openings. A wall surrounds each opening in the body thereby forming a cell culture well with a portion of the membrane functioning as the floor of the well. When a pressure differential is applied to the underside of the membrane, the membrane stretches, inducing strain on cells cultured thereon. The cell culture plate is sealingly positioned within a recess of a baseplate.

An object of the present invention is to provide a device that allows for an economic use of samples and that may be used in a variety of different microscopy applications. The object is achieved by the device having the features of claim 1.

Advantageous embodiments are described in the dependent claims.

According to claim 1, a sample holder assembly for optical microscopy comprises at least the following components:
- A first member having a first opening on a first side of the first member, and a circumferential wall portion laterally enclosing a first volume comprising the first opening, wherein the circumferential wall portion of the first member has an inward facing side that faces toward the first volume and an outward facing side opposite the inward facing side,
- A second member having a first opening on a first side of the second member, and a circumferential wall portion laterally enclosing a second volume, wherein the circumferential wall portion of the second member has an inward facing side facing toward the second volume and particularly an outward facing side opposite the inward facing side,

wherein a shape of the outward facing wall portion of the wall portion of the first member is formed complementary to the inward facing side of the wall portion of the second member, such that the wall portion of the first member can be inserted, particularly along an insert direction particularly from a second opening of the second member in the second volume, such that the first volume is at least partially comprised in the second volume, particularly such that the first volume coincides at least partially with the second volume, particularly such that the first openings of the first member and the second member coincide or essentially coincide, particularly wherein the first and the second opening extend along parallel planes,
   - A deformable transparent membrane having a first side for supporting a sample, wherein
the sample holder assembly is configured to adopt an assembled state, wherein in the assembled state, the membrane extends between the outward facing side of the wall portion of the first member, the inward facing side of the wall portion of second member, and covers the first opening of the first member, thereby forming a liquid-tight seal between the wall portions of the first and the second member and transparent imaging portion for microscopy at the first opening of the first member.

The invention is characterize din that the wall portion of the second member comprises at least two membrane-shaping portions that protrude or recede from a plane comprising the first opening of the second member, particularly wherein the membrane shaping portions are arranged opposite of each other, wherein the wall portion of the first member comprises at least two membrane-shaping portions formed complementary to the membrane-shaping portions of the second member, particularly wherein the membrane shaping portions of the first member are arranged opposite from each other corresponding to the membrane shaping portions of the second member, wherein in the assembled state of the sample holder assembly, the membrane is arranged between the complementary formed membrane-shaping portions of the first and the second member such that the membrane forms a non-even portion particularly extending through or across the first opening of the first member between the particularly oppositely arranged membrane shaping portions, wherein the non-even portion protrudes or recedes from a plane particularly completely comprising the first opening of the first member.

Particularly, the first member is arranged on a top side of the sample holder system, when the sample holder assembly is in use and arranged in the intended orientation. Accordingly, the second member may be arranged as the bottom member.

The first and the second member are formed correspondingly such that the wall portion of the first member and the wall portion of the second member fit complementary to each other. This way, the wall portion of the first member may be inserted in the second volume formed by the wall portion of the second member with the membrane arranged between the wall portions such that two effects are achieved:
1) The membrane is fixed relative to the assembled sample holder assembly,
2) A gap between the wall portions of the first member and the second member is sealed liquid-tight by the membrane such that a liquid tight sample volume is created that extends within the first volume onwards form the first side of the membrane.

The sample volume is circumferentially enclosed by the wall portion of the first member and covered by the membrane that extends - in the assembled state of the sample holder assembly - across the first opening of the first member. The membrane-shaping portions of the first and the second member are particularly arranged on opposing sides in the first opening of the member. Particularly, in the assembled state, the membrane-shaping portions of the first and the second member are arranged pairwise complementary to each other.

The membrane-shaping portions force the membrane to form the non-even membrane portion, particularly wherein said non-even portion protrudes only inward the first volume and not outwards, such that the membrane extends either within the plane comprising the first opening of the first member or protrudes away into the first volume. The non-even portion may be exploited for various purposes. For example, in digital light sheet microscopy the non-even portion may be formed such that it is suitable for said microscopy modality.

Alternatively, or additionally, the non-even portions may be used to one or more compartments in the sample volume, each compartment being fluidically disconnect form each other.

According to another embodiment of the invention, the non-even portion may protrude outward of the first volume forming a recess in which a sample may be arranged.

It is noted that the shape of non-even portion is particularly not solely caused by means of gravitational pull on the membrane but is a result of a lasting plastic deformability of the membrane.

According to another embodiment of the invention, the membrane-shaping portions are formed by a protruding element on the side of the wall portions that face toward the first or second volume.

The membrane-shaping portions may comprise a cross-sectional shape along a cross-section extending along the z-axis that reflects and imprints the shape of the membrane's non-even portion across the first opening.

According to another embodiment of the invention, the first opening of the first and/or of the second member extend(s) within a plane. Particularly, the first opening and/or the second opening is/are defined by a third side of the wall portion of the first or the second member respectively, wherein said third side forms a rim portion of the respective opening and may be comprised in the said plane.

For reasons of illustration, a coordinate system may be associated to the sample holder assembly, wherein a lateral direction is for example given by an x- and y-axis or by a radial and angular coordinate, and wherein a third direction, e.g. the z-axis.

When associated the coordinate system to the sample holder assembly, the openings of the first and/or the second member particularly are parallel to the x-y plane. The wall portions extend along the z-axis, such that the first and the second volume are formed. The opening of the first member may be a circular, or rectangular opening, but virtually any other shape is conceivable. Particularly, deviations of the geometric shape of the opening may arise due to additional elements comprised by the sample holder assembly.

According to another embodiment of the invention, the wall portion of the first member has a circular or a rectangular cross-section along a plane parallel to the x-y plane.

According to another embodiment of the invention, the wall portion of the second member has a circular or a rectangular cross-section along a plane parallel to the x-y plane.

According to another embodiment of the invention, the transparent member comprises at least one transparent portion, particularly wherein in the assembled state of the sample holder system the transparent portion is arranged at the first opening of the first member.

According to another embodiment of the invention, the first and/or the second member is/are formed by a rigid material such that the first and/or the second member provide a stable framework for the membrane and the sample holder assembly.

According to another embodiment of the invention, in the assembled state the first openings of the first member and the second member coincide, particularly such that the openings are comprised in the same plane.

According to another embodiment of the invention, the membrane is a continuous membrane.

The membrane is particularly deformable, more particularly plastically deformable membrane. The membrane further comprises an elastic deformable component.

The first side of the membrane is intended to carry the sample.

The sample holder assembly is configured to be repeatedly assembled and disassembled that is the first member and the second member put together and removed from each other repeatedly. The sample holder assembly therefore provides a multi-use system, that can be used many times, for example by exchanging the membrane after use, while reusing the first and the second member. The sample holder assembly does not require gluing or welding of components in order to create a liquid-tight sample volume.

Thus, the sample holder assembly may be configured for multi-use or one-time use, depending on the application.

Particularly when designed for one-time use, the first and the second member may comprise corresponding locking members that form a positive locking between the first and the second member, when the sample holder assembly is brought in the assembled state, such that the first and the second member cannot be disengaged when the locking members are engaged with each other.

According to another embodiment of the invention, the first and/or the second wall portions are formed from a metal or a polymer.

According to another embodiment of the invention, the first and/or the second member are formed from a metal or a polymer.

According to another embodiment of the invention, in the assembled state the membrane is friction-locked between the wall portions of the first and the second member, particularly wherein the membrane is fixed in the sample holder assembly only by means of the frictional connection between the membrane and the wall portions of the first and the second member. This allows for the omission of glue in the sample holder assembly.

Friction locking the membrane between the wall portions also allows for a particularly facile and simple geometry for a sample holder assembly that can be used flexible and repeatedly.

According to another embodiment of the invention, the second member comprises a second opening opposite the first opening, the second opening being connected by the wall portion of the second member to the first opening, wherein the assembly is configured to adopt an unassembled state in which the membrane covers, and particularly is arranged with its second side on the second opening and wherein the sample holder assembly is configured to transition from the unassembled state to the assembled state by inserting, e.g. by a user, the wall portion of the first member in the second opening of the second member and moving the first member toward the first opening of the second member, thereby moving the membrane from the second opening to the first opening of the second member, thereby forming the liquid-tight seal between the wall portions of the first and the second member, particularly wherein when the sample holder assembly is in its assembled state, the first opening of the first and the second member may be close to each other, particularly wherein the sample holder assembly is configured to transition repeatedly from the assembled state to the unassembled state and vice versa.

This embodiment discloses a way of assembling the sample holder assembly. In simplifying words, the first member is used to push the membrane upon assembly of the sample holder assembly toward the first opening of the second member and simultaneously form the liquid-tight seal between the wall portions.

According to another embodiment of the invention, the first member and the second member are configured to form the assembled sample holder assembly, by means of a push-mechanism, particularly wherein the first member is not connected to the second member, by means of a screw- or interlock mechanism. Particularly, the first and the second member are not positively locked and not integrally formed with each other.

According to another embodiment of the invention, the membrane-shaping portions of the second member have a sawtooth-shaped cross-section particularly along the z-axis such that the membrane protrudes at the non-even portion toward the second volume at a wedge angle enforced by the membrane shaping portions of the second member.

The wedge angle is defined by the steepness of the sawtooth, i.e. the steeper the sawtooth, that larger the wedge angle.

In the assembled state, the non-even portion protrudes toward the first volume.

The cross-sectional shape of the membrane-shaping portions according to this embodiment has a saw-tooth shape, particularly wherein the membrane-shaping portions themselves are wedge-shaped.

This embodiment may be particularly useful in digital light sheet microscopy, as it provides an inclined plane relative to a horizontal plane, e.g. the x-y plane, on which a sample may be arranged for imaging with a digital light sheet microscope, such as the Leica TCS SP8 DLS.

According to another embodiment of the invention, the sample holder assembly further comprises:
- a first, particularly not connected with the sample holder assembly, movable shape support member that fits between the at least two membrane shaping portions of the first member, wherein the first shape support member has a membrane facing side that has the same cross-sectional shape as the membrane-shaping portion of the first member,
- a second, particularly not connected with the sample holder assembly, movable shape support member that fits between at least two membrane shaping portions of the second member, wherein the second membrane shape support member has a membrane facing side that has the same cross-sectional shape as the membrane-shaping portion of the second member, wherein the first and the second membrane support members, when arranged between the membrane shaping portions, extend along the non-even portion of the membrane from opposite sides of the membrane with their corresponding membrane facing sides oriented toward the membrane, such that the membrane is arranged between the first and second membrane shape support member, such that a membrane contour along the non-even portion of the membrane is formed to correspond to the cross-sectional shape of the membrane shaping portions of the first and the second member across the first opening, particularly such that the membrane maintains said shape across the first opening even when the shape support members are removed.

The shape support members provide an efficient means to enforce the cross-sectional shape form the non-even membrane portions over the extend of the non-even portion across the first opening.

This embodiment allows for a creation of the non-even membrane portion with a precise and predefined shape that lasts even when the support members are removed from the membrane. This is particularly achieved by the plastic deformability of the membrane.

In simplifying words, the support members act as stamps with a predefined contour for imprinting the predefined shape in the membrane to form the non-even membrane portion.

According to another embodiment of the invention, the non-even membrane portion has a height above the plane comprising the first opening, wherein said height is at least 1 mm, particularly larger than 5 mm, such that the non-even membrane portion separates the second volume in a first and a second compartment, which are fluidically disconnect.

This embodiment allows a formation of at least two disconnect compartments, in which for example different samples may be cultured.

Alternatively, the non-even membrane portion forms a complementary recess, such that a recess is formed in which a sample may be cultured.

According to another embodiment of the invention, the assembly comprises a pusher element having a body, wherein the body is shaped such that at least in one orientation of the body, the body fits in second volume, such that the body can be inserted in the second volume from the side of the second opening of the second member, wherein the pusher element is configured to push the membrane covering the second opening in the unassembled state of the assembly toward the first opening of the second member, such that the membrane adopts a pre-shaped form, such that when inserting the wall portion of the first member in the second volume, membrane deformation forces are reduced due to the pre-shaped form.

The pusher element may be used to pre-form the membrane, when the sample holder assembly is in the unassembled state, by allowing to push the membrane from the second opening of the second member toward the first opening of the second member. This embodiment provides a means for assembling the sample holder assembly such that a force necessary to assemble the sample holder assembly by moving the first member toward the first opening of the second member is reduced, as the membrane is already in a pre-formed configuration provided by the pusher element, wherein from the pre-formed configuration a lower force is required in order to arrive in the assembled state of the sample holder assembly.

The pusher element for example comprises rigid body, that for example has a piston shape complementary to the second volume. For example, the body has a continuous and smooth surface such that tear of the membrane is reduced.

According to another embodiment of the invention, the sample holder assembly comprises a plurality of the first members that are integrally formed with each other and a plurality of the second members that are integrally formed with each other and wherein the first and the second members have spatially corresponding wall portions such that an array of corresponding first and second members is formed, particularly wherein in the assembled state, the membrane extends between the outward facing side of the wall portions of the first members, the inward facing side of the wall portions of second members, and covers the first openings of the first members, thereby forming a liquid-tight seal between the wall portions of the first and the second members and a transparent imaging portion and particularly a sample support for microscopy at the first openings of the first member.

This embodiment provides an array of sample volumes for imaging a plurality of different samples.

According to another embodiment of the invention, the pusher element has a plurality of integrally formed bodies arranged correspondingly to the integrally formed second members, such that in the unassembled state of the assembly the pusher element is configured to simultaneously push the membrane in the second volumes formed by the wall portions of the second members, such that the membrane adopts a pre-shaped form, such that when inserting the wall portions of the first members in the second volumes, membrane deformation forces are reduced due to the pre-shaped form.

This embodiment allows provides a means for assembly of the sample holder assembly, as elaborated above for the pusher element having one body. The bodies may have the complementary shapes of the second volumes.

According to another embodiment of the invention, the membrane is flexible and plastically deformable, such the membrane maintains a shape, particularly a shape of the non-even portion, particularly in the absence of a supporting means, such as the first and/or the second support member.

According to another embodiment of the invention, the membrane has a refractive index that corresponds to the refractive index of water within a 20% margin of deviation, particularly wherein the refractive index is in the range of 1.333 ± 0.05.

According to another embodiment of the invention, the membrane comprises or consists of fluorinated ethylene propylene (FEP, CAS-Nr: 25067-11-2), polytetrafluoroethylene (PTFE, CAS-Nr: 9002-84-0), ethylene tetrafluoroethylene (ETFE, CAS-Nr: 25038-71-5).

According to a second aspect of the invention, the problem according to the invention is advantageously solved by a microscope comprising the sample holder assembly according to any of the preceding embodiments. For example, the microscope may comprise a digital light sheet module. The microscope may further or alternatively comprise a confocal and/or a wide-field imaging module. In general, the microscope may be configured to perform optical microscopy, and or superresolution optical microscopy.

The versatile possible configurations render the sample holder assembly flexible to adopt preferred shapes for any of the imaging modalities of the microscope, as elaborated in previous embodiments.

According to a third aspect of the invention, a method for imaging a sample with the assembling a sample holder assembly according to the previous embodiments is disclosed, wherein the method comprises at least the steps of:
- Arranging the membrane with a second side of the membrane that is opposite the first side of the membrane on the second opening of the second member or the second openings of the second members,
- Particularly pushing the membrane towards the first opening(s) of the second member(s) into the second volume(s) by means of the pusher element,
- Inserting the wall portion(s) of the first member(s) into the second volume(s) such that the membrane covers the first opening (s) and the liquid tight seal is formed between each of the wall portions of the first and the second member(s), thereby creating a sample imaging volume in the first volume(s),
- Particularly shaping the non-even membrane portion across the first opening of the first member by means of the first and second movable shape support member.
- Removing the first and second movable shape support member form the sample holder assembly,
- Particularly growing the sample on the first side of the membrane or arranging the sample on the first side of the membrane in the first volume,
- Image the sample.

It is noted that the steps do not have to be executed in the order provided by may be adjusted.

Features, embodiments and definitions disclosed in the context of embodiments relating to the sample holder assembly or the microscope apply the method according to the invention and vice versa.

### Figure description and examples

Particularly, exemplary embodiments are described below in conjunction with the Figures. The Figures are appended to the claims and are accompanied by text explaining individual features of the shown embodiments and aspects of the present invention. Each individual feature shown in the Figures and/or mentioned in said text of the Figures may be incorporated (also in an isolated fashion) into a claim relating to the device according to the present invention.

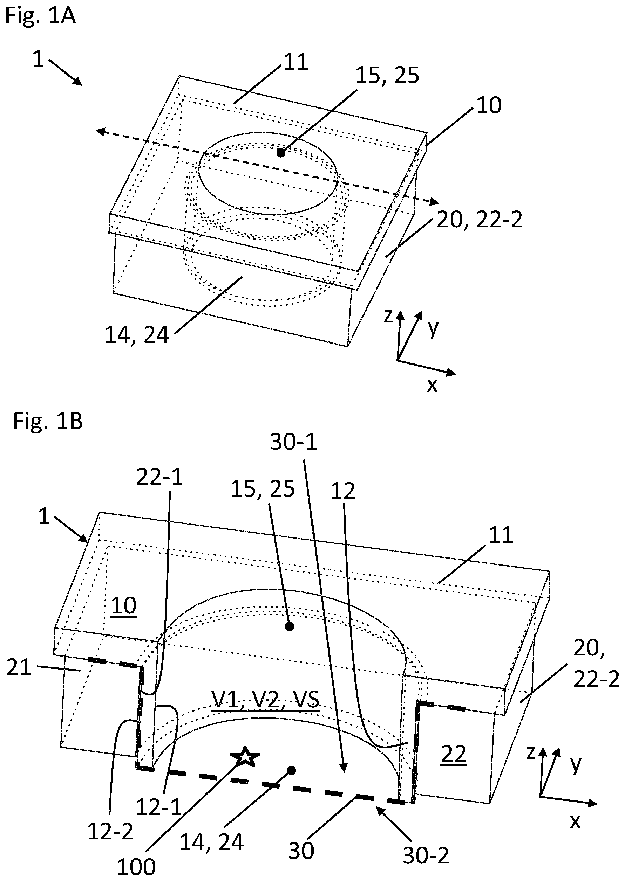

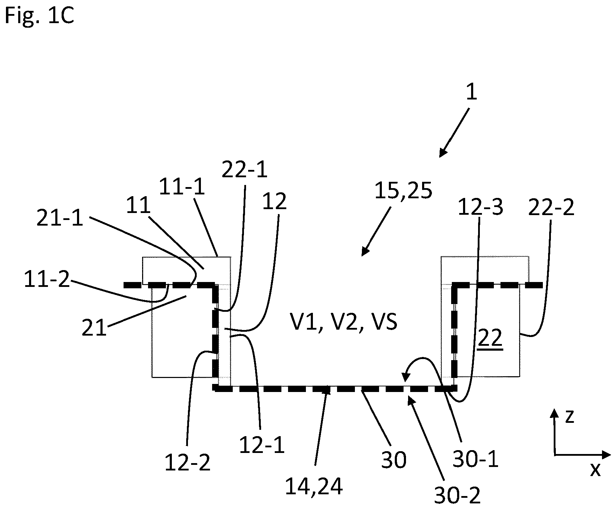

In Fig. 1A to 1C shows various schematic views of a sample holder assembly, that is not part of the invention. Fig. 1A shows a three-dimensional perspective view of a sample holder assembly 1, Fig 1B shows a perspective cross-section and Fig. 1C shows a cross-section of the sample holder assembly 1. The sample holder assembly 1 comprises a first member 10 and a second member 20. A cartesian coordinate system having an x-, y- and z-axis as depicted is associated to the sample holder assembly 1. The first member 10 is formed as a top member, wherein the second member 20 is formed as a bottom member. The first member 10 comprises a rigid top portion 11 that extends in a lateral plane, particularly in a horizontal plane, parallel to the x- and the y-axis. Integrally formed with the top portion 11 is a wall portion 12 that extends orthogonally away from the top portion 11, particularly wherein the wall portion 12 extends along the z-axis and encloses a first volume V1 that in this embodiment has a cylindrical shape. The wall portion 12 of the first member 10 has a first side 12-1 facing toward the first volume V1 and a second side 12-2- facing in the opposite direction, i.e. away from the first volume V1. The wall portion 12 of the first member 10 has an annular cross-section along the lateral plane. The wall portion 12 of the first member 10 ends at and delimits a first opening 14 of the first member 10, wherein said opening 14 is spaced apart from the top portion 11 of the first member 10. The first opening 14 limits the first volume V1 towards a bottom side, i.e. toward a side where in the assembled state (cf. Fig. 1B) of the sample holder assembly 1 the membrane 30 is located. The wall portion 12 of the first member 10 further comprises a second opening 15 that is comprised by the top portion 11. The second opening 15 is arranged opposite the first opening 14. The first and the second openings 14, 15 are connected by the wall portion12. It is noted that the second opening 15 is an optional feature of the first member 10. The second opening 15 might be helpful to access a sample 100 arranged at the first opening 14 on a first side of the membrane 30-1 that face toward the first volume V1. The membrane 30 has a second side 30-1 that faces in the opposite direction than the first side 30-1 of the membrane 30.

The top portion 11 has a square shape in the lateral plane, which provides compatibility to a larger variety of microscopes sample holder systems configured to receive a sample holder. It is noted that other shapes of the top portion 11 are well within the intended scope of protection.

The sample holder assembly 1 of Fig. 1 further comprises a second member 20 that comprises a top portion 21 that extends in the lateral plane and provides a support and a hard stop for the top portion of the first member 10 during and after assembly of the sample holder assembly 1. The second member 20 comprises a wall portion 22 that extends orthogonally to the top portion 21. The wall portion 22 is integrally formed with the top portion 21 and laterally encloses a second volume V2 that has a cylindrical shape in this example. The wall portion 22 of the second member 20 has a first side 22-1 facing toward, i.e. inward the second volume V2. A second side facing 22-2 in the opposite direction is formed at an outer surface of the second member 20.

The second volume V2 is limited by a first opening 24 of the wall portion 22 of the second member 20 and a second opening 25. The first and the second openings 24, 25 limit the cylindrical second volume V2, wherein the second opening 25 is arranged in the plane of the top portion 21 of the second member 20. A cross-sectional diameter of the circumferential second outward facing side 12-2 of the circumferential wall portion 12 of the first member 10 is slightly smaller than a cross-sectional diameter of the circumferential first inward facing side 22-1 of the circumferential wall portion 22 of the second member 20. This way, the wall portion 12 of the first member 10, and thus the first volume V1, can be inserted via the second opening 25 of the second member 20 into the second volume V2. Particularly, the wall portion 12 of the first member 10 can be inserted until the top portion 11 of the first member 10 meets the hard stop formed by the top portion 21 of the second member 20. In order to provide a good fit for the first and the second member 10, 20, the top portion 11 of the first member 10 has a bottom surface 11-2 that is planar and the top portion 21 of the second member 20 has a planar top surface 21-1, that match the planar bottom surface of the first portion 11.

The sample holder assembly 1 comprises a transparent plastically deformable membrane 30. The membrane may be deformed by an external force and adopt and maintain an altered shape even when the external force has ceased.

The sample holder assembly 1 is configured to adopt an assembled state in which the wall portion 11 of the first member 10, i.e. the first volume V1, is inserted in the second volume V2 of the second member 20 (the assembled state is shown in Fig. 1A to 1C). Further, in the assembled state, the membrane 30 is arranged between the second outward facing side 12-2 of the first member 10 and the first inward facing side 22-1 of the wall portion 22 of the second member 20. In the assembled state of the sample holder assembly 1, the membrane 30 covers the first opening 14 of the first member 10 completely and forms a sample volume VS comprised in, particularly coinciding with in the first volume. Thus, at the first opening 14, the membrane 30 extends over a third side 12-3 of the wall portion 12 of the first member 10. Said third side 13-2 particularly faces toward the first opening 14. The membrane 30 further extends between the planar bottom surface 11-2 of the top portion 11 of the first member 10 and the top surface of the top portion 21 of the second member 20.

In the assembled state the membrane 30 is friction-locked between the first member 10 and the second member 20, particularly between the wall portions 12, 22 of the first member 10 and the second member 20. The fit between the outward facing second side 12-2 of the wall portion 12 of the first member 10 and the inward facing first side 22-1 of the wall portion 22 of the second member 20 therefore is adjusted just so, that when the membrane 30 is arranged between the first member 10 and the second member 20 the membrane is friction-locked. The sample holder assembly 1, due to the specific course of the membrane between the first member 10 and the second member 20 provides a liquid-tight sample volume VS, such that no leaking of any fluid through the first opening 14 of the first member 10 might take place.

The membrane 30 in the assembled state of the sample holder assembly 1 has a first side 30-1 facing toward the first volume V1 on which the sample 100 may be arranged. Imaging can be performed on/from both sides 30-1, 30-2 of the membrane 30. As the refractive index of the membrane 30 is preferably chosen close to the refractive index of water or to the refractive index of an immersion oil for a microscope objective, optical aberrations are kept at a minimum. Further, the membrane 30 in this example extends essentially planar across the first opening 14 of the first member 10.

For assembly, the first member 10 is essentially pushed downward with its first opening 14 toward the first opening 24 of the second member 20 until the bottom surface 11-2 of the top portion 11 of the first member 10 is hard stopped by the top surface 21-1 of the top portion 21 of the second member 20. In the assembled state, the first openings 14, 24 of the first member 10 and the second member 20 may coincide. Under close inspection of the schematic Fig 1A, it can be seen that the first openings 14, 24 do not exactly coincide. Both design options are suitable.

The features, reference signs and relations between the components described for the embodiment shown in Fig. 1 apply in an analogous fashion to the examples illustrated in the following figures as long as not stated otherwise.

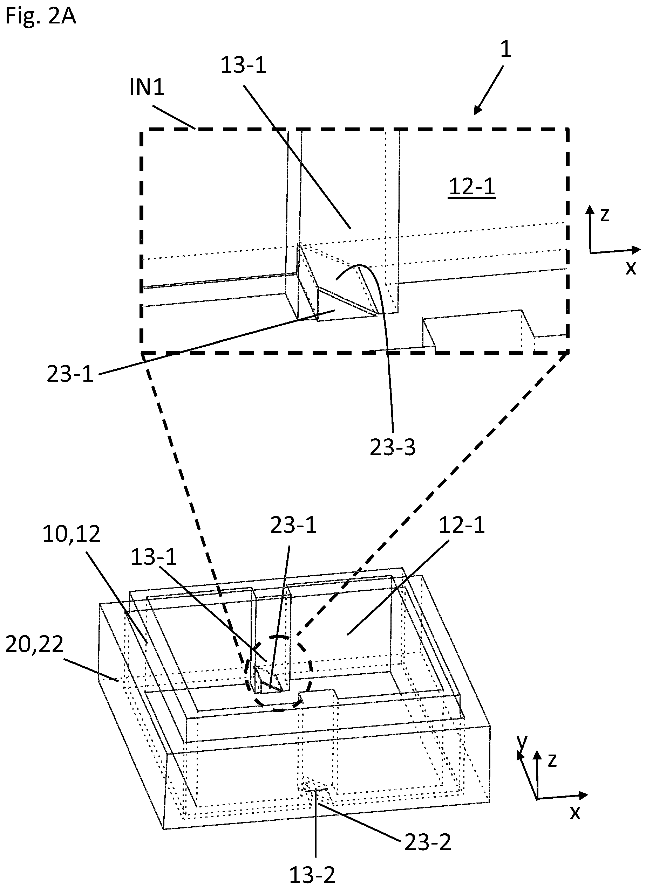

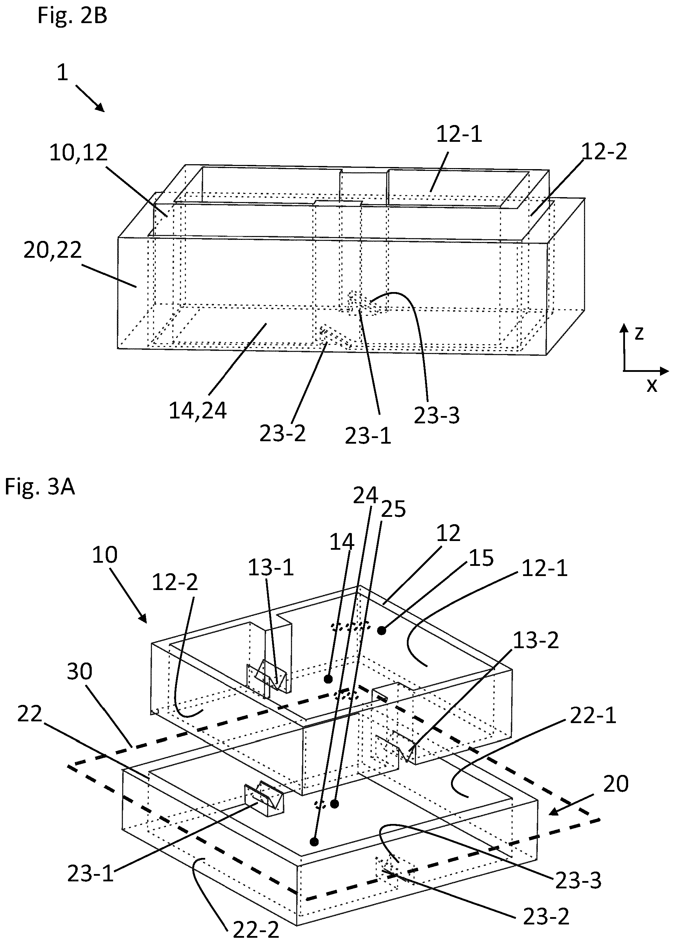

Fig. 2A to Fig. 2C show various views of an exemplary embodiment of the invention. Fig. 2A shows a three-dimensional perspective view of sample holder assembly 1, wherein the inset IN1 shows a magnified view from the detail encircled by the circle in broken lines in Fig. 2A. Fig. 2B shows a slight variant (rectangular instead of square geometry of the sample holder assembly) of the embodiment shown in Fig. 2A.

The sample holder assembly 1 of Fig. 2A and 2B has a cubic or cuboid geometry of the sample volume VS. The wall portions 12, 22 of the first member 10 and the second member 20 have a square or a rectangular cross-section in the lateral plane, providing an essentially square / rectangular first opening 14, 24 to the first member 10 and the second member 20. Also, the second openings 15, 25 are square / rectangular. The outwards facing second side 12-2 of the wall portion 12 of the first member 10 is complementary to the inward facing first side 22-1 of the wall portion 22 of the second member 20, such that the wall portion 12 and with it, the first volume V1, can be inserted in the second volume V2 of the second member 20, having the wall portions 12, 22 closely fit their respective complementary contours 12-2, 22-1.

The first member 10 and the second member 20 do not comprise top portions, but essentially consist of the wall portions 12, 22. In the assembled state of the sample holder assembly 1 the membrane 30 is friction-locked between the outward facing second side 12-2 of the wall portion 12 of the first member 10 and the inward facing side first side 22-1 of the wall portion 22 of the second member 20 and covers the first opening 14 of the first member 10, such that a sample volume is closed liquid tight toward the first opening 14 (not explicitly depicted).

The second member 20 comprises two membrane shaping portions 23-1, 23-2 that are arranged on the inward facing first side 22-2 of the wall portion 22 of the second member 20. The membrane shaping portions 23-1, 23-2 protrude away from the first side 22-1 inward the second volume V2 of the second member 20. The membrane shaping portions 23-1, 23-2 are arranged opposite from each other on the wall portion 22 of the second member 20 at the first opening 24 of the second member 20.

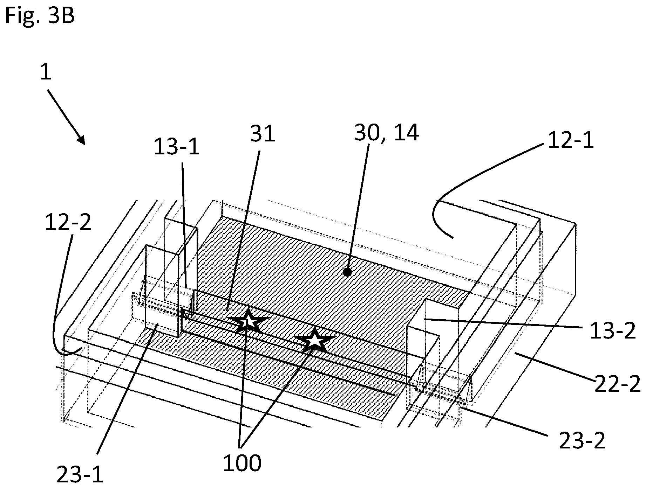

The membrane shaping portions 23-1, 23-2 each are formed as a wedge. The wedges have a rising face 23-3 that point along the same direction for both membrane shaping portions 23-1, 23-2. The membrane shaping portions 23-1, 23-2 are identical in shape. In the assembled state, the membrane 30 extends over the first opening 14 of the first member 10 and follows the rising faces 23-3 of the membrane shaping portions 23-1, 23-2 such that the membrane extends partially out of the plane defined by the first opening 14 inward the first volume V1 (the membrane is not shown in this example). The membrane shaping portions 23-1, 23-2 are configured and adapted to force the membrane to form a non-even portion 31 that extends between the membrane shaping portion across the first opening 14 (cf. e.g. Fig. 3B, Fig. 4 and Fig. 6B). Wherein said non-even portion 31 adopts the same contour as the membrane shaping portions 23-1, 23-2.

In the assembled state, the first member 10 comprises two membrane shaping portions 13-1, 13-2 that have a complementary surface to the rising face 23-3 and the sawtooth shape of the wedges 23-1, 23-2, such that the membrane shaping portions 13-1, 13-2 of the first member fit to the membrane shaping portions 23-1, 23-2 of the second member 20 in the assembled state. Thus, the membrane shaping portions 13-1, 13-2 are arranged at the corresponding locations at the inward facing wall portion 12-1 of the first member 10. Here, the membrane shaping portions 13-1, 13-2 of the first member 10 are formed as protruding elements having a recess at a side facing toward the first opening 14 that is formed complementary to the sawtooth surface 23-3 of the membrane shaping portions 23-1, 23-2 of the second member 20.

Therefore, in the assembled state, the membrane 30 is fixed by means of friction-locking between the corresponding membrane shaping portions 13-1, 23-1 and 13-2, 23-2, and thereby forced to assume the sawtooth contour along the non-even membrane portion 31.

The sample holder assembly 1 according to this example allows creating an imaging surface for microscopy modes that require a tilted or non-even surface. Moreover, this way, the sample holder assembly 1 can provide compartments that are fluidically disconnect by the non-even membrane portion 31 from each other in the sample volume.

The membrane shaping portions 23-1, 23-2 also serve as a hard stop for the first member 10 such the sample holder assembly 1 can be assembled precisely.

Wall portion thickness is usually in the range of several millimeters.

Fig. 3A and 3B show a variation of the sample holder assembly 1 of Fig. 2, having membrane shaping portions 23-1, 23-2 (of the second member 20) that cause the membrane 30 to form a protruding wall section having a V-shaped recess on top. The V-shaped recess may be used for arranging a sample centrally in a bottom portion of the V-shaped recess. The complementary membrane shaping portion 13-1, 13-2 of the first member 10 has a corresponding M-shaped recess, that complements the wall-section with the V-shaped recess formed by the membrane shaping portion 23-1, 23-2 of the second member 20. The membrane shaping portions 23-1, 23-2 serve as a hard stop also in this example. In Fig. 3A the sample holder assembly 1 is shown in an unassembled state, so that the membrane shaping portions 23-1, 23-2, 13-1, 13-2 can be seen separately. By placing the membrane 30 on top, i.e. on the second opening 25 of the wall portion 22 of the second member 20, and subsequently pushing the first member 10 downwards towards the first opening 24 of the second member 20, the membrane 30 is forced into its final shape, where it provides a liquid-tight seal between the first member 10 and the second member 20 as well as an imaging platform for a sample. Moreover, the membrane shaping portions 13-1, 13-2, 23-1, 23-2 force the membrane to adopt a locally non-even contour 31 that protrudes toward the first volume V1 forming a wall section 31 having the V-shaped recess. This non-even shape of the non-even portion 31 may be advantageously used for digital light-sheet imaging. The assembled state is shown in Fig. 3B, where a plurality of samples 100 is arranged in the V-shaped recess of the wall section formed by the non-even portion 31.

The first volume V1 may be filled with a solution for cultivating the samples 100.

The sample holder assembly 1 of Fig. 3 is so large and comprises second openings 15, 25 that are sufficiently large so that a microscope optics can be placed inside the first volume V1.

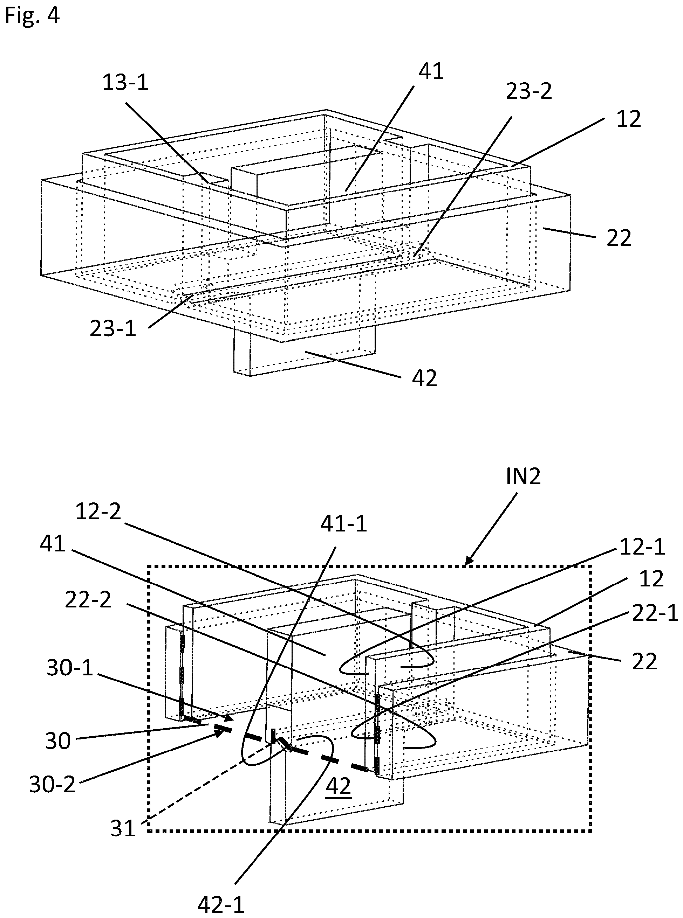

Fig. 4 shows essentially the example of Fig. 2A, however with the additional components of the shape support members 41, 42 that can be arranged on the first 30-1 and the opposite second side 30-2 of the membrane 30 (shown in inset IN2). The shape support members 41, 42 comprise a first shape support member 41 and a second shape support member 42, which both have a first side 41-1, 42-1 facing toward the membrane 30, particularly wherein the first side 41-1 of the first shape support member 41 faces the first side 30-1 of the membrane and the first side 42-1 of the second shape support member 42 faces the second side 30-2 of the membrane, wherein said sides 41-1, 42-1 have complementary shapes and correspond to the shapes of the membrane shaping portions 13-1, 13-2 and 23-1,23-2 such that a cross-section of the membrane shaping portion is continued across the non-even portion 31 of the membrane 30. In this sense, the support members 41, 42 serve as stamping means to imprint and support the membrane shape intended by the membrane shaping portions across the first opening 14. For this purpose, the support members 41, 42 can be inserted and removed from the sample holder assembly 1 before imaging. When the support members 41, 42 are removed, the membrane 30, due to its mechanical properties maintains the imprinted shape, i.e. the membrane 30 is plastically deformable. The inset IN2 shows a detail of the cross-section indicated by the broken line.

The first support member 41 may be inserted through the second opening 15, 25 and faces the first side 30-1 of the membrane 30, wherein the second support member 42 may be arranged at the second side 30-2 of the membrane 30 and faces toward the first volume V1. In the intended configuration of the support members 41, 42, the support members 41, 42 are arranged at the same positions as the membrane shaping portions 23-1, 23-2, 13-1, 13-2.

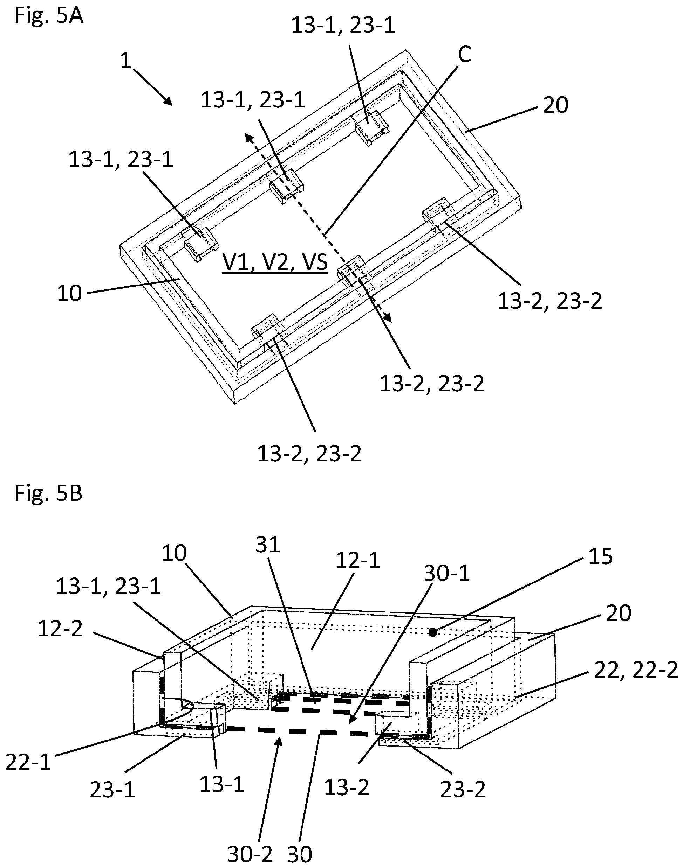

Fig. 5A and 5B show yet another example of the sample holder assembly 1. In the example shown in Fig. 5A a top view of the sample holder assembly 1 is shown, wherein in Fig. 5B a cross-sectional perspective view is shown as indicated by the broken line C in Fig. 5A.

In addition to the previous examples, the sample holder assembly 1 comprises a plurality of pairwise arranged membrane shaping portions 23-1, 23-2, 13-1, 13-2, such that a plurality of non-even membrane portions 31 can be created across the first opening 14. In this example, the non-even membrane portions 31 form cuboid wall sections extending across the first opening 14. The membrane shaping portions 13-1, 13-2, 23-1, 23-2 are correspondingly shaped, as elaborate above.

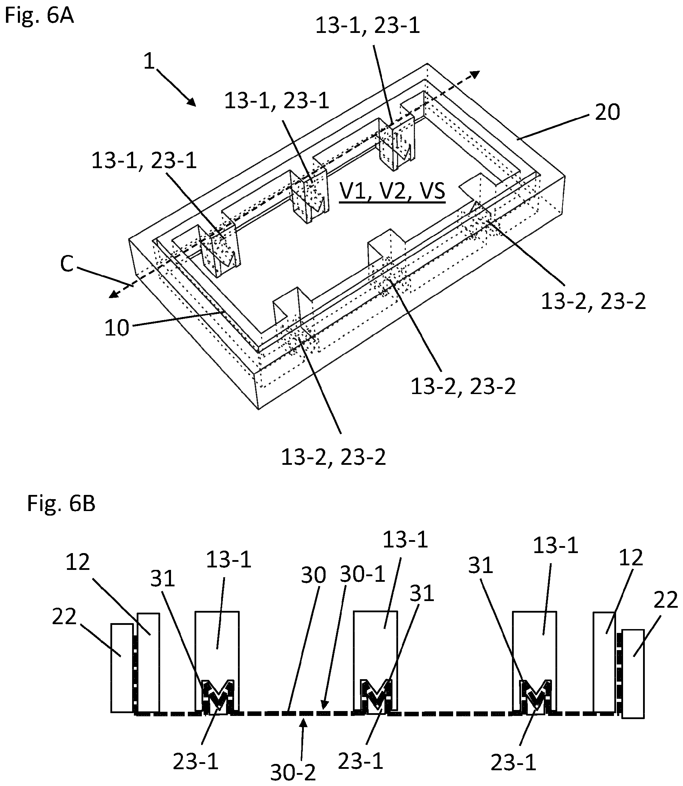

A similar example to Fig. 5 is shown Fig. 6, where a plurality of wall sections comprising a V-shaped recess on top are formed by the plurality of non-even membrane portions 31 that are created by a plurality of pairwise arranged membrane shaping portions 13-1, 13-2, 23-1- 23-2. Fig. 6 shows a three-dimensional perspective view of the sample holder assembly 1, wherein Fig. 6B shows a vertical cross-section across the broken line C indicated in Fig. 6A.

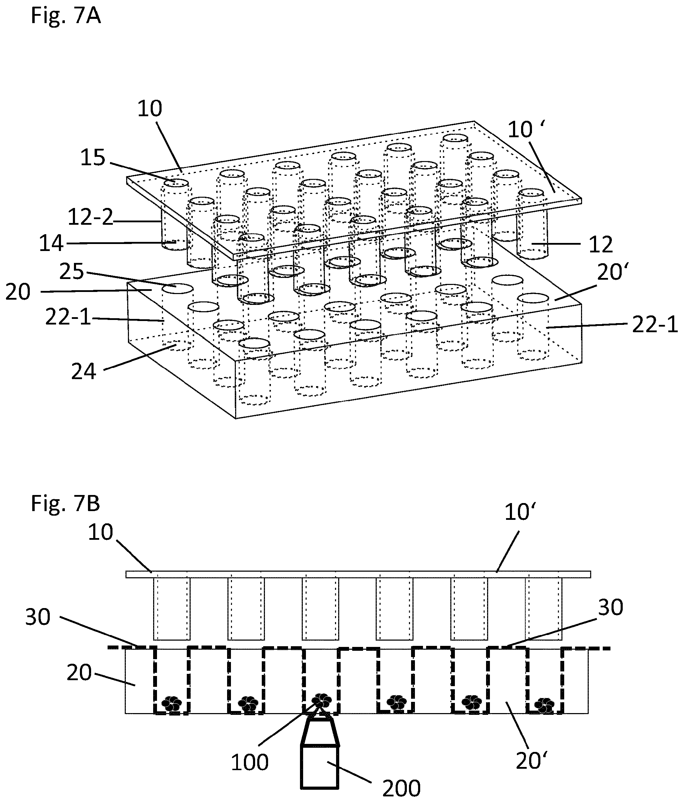

Fig. 7A and 7B depicts an example in which the sample holder assembly 1 forms an array having a plurality of sample volumes. For this purpose, the sample holder assembly 1 comprises a plurality of first members 10, 10' that are integrally formed with each other. Therefore, the sample holder assembly 1 comprises a plurality of wall portions 12 that, in this example, each form a cylindrical sample volume. The array comprises 24 sample volumes arranged in 6 rows and 4 columns. Correspondingly, the second members 20, 20' are arranged in 6 rows and 4 columns each and are integrally formed with each other as well. Therefore, by moving the first volumes V1 of the first members 10, 10' into the second volumes V2 of the second members 20, 20', the assembled state of the sample holder assembly 1 is adopted.

The membrane 30 extends between the wall portions of the first and the second members 10, 10', 20, 20'.

Fig. 7A shows the sample holder assembly 1 in the unassembled start, wherein Fig. 7B shows the sample holder assembly 1 in an exploded cross-sectional view, with the membrane 30 being arranged as in the assembled state.

Imaging of samples 100 comprised in the sample volumes may be facilitated by an appropriate imaging optics 200

## Claims

1. A sample holder assembly (1) for microscopy comprising at least the following components:
- A first member (10) having a first opening (14) on a first side of the first member (10), and a circumferential wall portion (12) laterally enclosing a first volume (V1) comprising the first opening (14), wherein the circumferential wall portion (12) of the first member (10) has an inward facing side (12-1) that faces toward the first volume (V1) and an outward facing side (12-2) opposite the inward facing side (12-1),
- A second member (20) having a first opening (24) at a first side of the second member (20), and a circumferential wall portion (22) laterally enclosing a second volume (V2), wherein the circumferential wall portion (22) of the second member (20) has an inward facing side (22-1) facing toward the second volume (V2), wherein a shape of the outward facing side (12-2) of the wall portion (12) of the first member (10) is complementary to the inward facing side (22-1) of the wall portion (22) of the second member (20), such that the wall portion (12) of the first member (10) can be inserted in the second volume (V2), such that the first volume (V1) is at least partially comprised by the second volume (V2),
- A transparent membrane (30) having a first side (30-1) for supporting a sample (100), wherein the sample holder assembly (1) is configured to adopt an assembled state, wherein in the assembled state, the membrane (30) extends between the outward facing side (12-2) of the wall portion (12) of the first member (10), the inward facing side (22-1) of the wall portion (22) of second member (20), and covers the first opening (14) of the first member (10), thereby forming a liquid-tight seal between the wall portions (12, 22) of the first and the second member (10, 20) and a transparent imaging portion for microscopy at the first opening (14) of the first member (10),
**characterized in that**
the wall portion (22) of the second member (20) comprises at least two membrane-shaping portions (23-1, 23-2) that protrude or recede from a plane comprising the first opening (24) of the second member (20), wherein the wall portion (12) of first member (10) comprises at least two membrane-shaping portions (13-1, 13-2) formed complementary to the membrane-shaping portions (23-1, 23-2) of the second member (20), wherein in the assembled state of the sample holder assembly (1), the membrane (30) is arranged between the complementary formed membrane-shaping portions (13-1, 13-2, 23-1, 23-2) such that the membrane (30) forms a non-even portion (31) between the two membrane shaping portions (13-1, 13-2, 23-1, 23-2), wherein the non-even portion (31) protrudes or recedes from a plane comprising the first opening (14) of the first member (10).

2. The sample holder assembly (1) according to claim 1, wherein in the assembled state the membrane (30) is friction-locked between the wall portions (12, 22) of the first and the second member (10, 20), particularly wherein the membrane (30) is fixed in the sample holder assembly (1) only by means of the frictional connection between the membrane (30) and the wall portions (12, 22) of the first and the second member (10, 20).

3. The sample holder assembly (1) according to any of the claims 1 or 2, wherein the second member (20) comprises a second opening (25) opposite the first opening (24), the second opening (25) being connected by the wall portion (22) of the second member (20), wherein the sample holder assembly (1) is configured to adopt an unassembled state, in which the membrane (30) covers the second opening (25) and wherein the sample holder assembly (1) is configured to transition from the unassembled state to the assembled state by inserting the wall portion (12) of the first member (10) in the second opening (25) and moving the first member (10) toward the first opening (24) of the second member (20), thereby moving the membrane (30) from the second opening (25) to the first opening (24) of the second member (20), thereby forming the liquid-tight seal between the wall portions (12, 22) of the first and the second member (10, 20), particularly wherein the sample holder assembly (1) is configured to transition repeatedly from assembled state to the unassembled state and vice versa.

4. The sample holder assembly (1) according to one of the preceding claims, wherein the membrane-shaping portions (23-1, 23-2) of the second member (20) have a sawtooth-shaped cross-section such that the membrane (30) protrudes at the non-even portion (31) toward the second volume (V2) at a wedge angle enforced by the membrane shaping portions (23-1, 23-2) of the second member (20).

5. The sample holder assembly (1) according to one of the preceding claims, wherein the membrane shaping portions (23-1, 23-2) of the second member (20) have a convex-shaped cross-section, a concave-shape cross-section, a V-shaped-cross-section, a U-shaped cross-section and/or a rectangular-shaped cross-section, such that in the assembled state of the sample holder assembly (1), the non-even portion (31) of the membrane (30) adopts the corresponding shape of the membrane shaping portions (23-1, 23-2).

6. The sample holder assembly (1) according to one of the preceding claims, wherein the sample holder assembly (1) further comprises:
- a first movable shape support member (41) that fits between the at least two membrane shaping portions (13-1, 13-2) of the first member (10), wherein the first shape support member (41) has a membrane facing side that has the same cross-sectional shape as the membrane-shaping portion (13-1, 13-2) of the first member (10),
- a second movable shape support member (42) that fits between the at least two membrane shaping portions (23-1, 23-2) of the second member (20), wherein the second membrane shape support member (42) has a membrane facing side that has the same cross-sectional shape as the membrane-shaping portion (23-1, 23-2) of the second member (20), wherein the first and the second membrane support members (41, 42) when arranged between the membrane shaping portions (13-1, 13-2, 23-1, 23-2) extend along the non-even portion (31) of the membrane (30) from opposite sides (30-1, 30-2) of the membrane (30) with their corresponding membrane facing sides oriented toward the membrane (30), such that the membrane (30) is arranged between the first and second membrane shape support member (41, 42), such that a membrane contour along the non-even portion (31) of the membrane (30) is formed to correspond to the cross-sectional shape of the membrane shaping portions (13-1, 13-2, 23-1, 23-2) of the first and the second member (10, 20) across the first opening (14) of the first member (10).

7. The sample holder assembly (1) according to one of the preceding claims, wherein the non-even membrane portion (31) has a height above the plane comprising the first opening (14), wherein said height is at least 1 mm, such that the non-even membrane portion (31) separates the sample volume (VS) in a first and a second compartment, which compartments are fluidically disconnect.

8. The sample holder assembly (1) according to any of the preceding claims, wherein the sample holder assembly (1) comprises a pusher element having a body, wherein the body is shaped such that at least in one orientation of the body, the body fits in second volume (V2), such that the body can be inserted in the second volume (V2) from the side of the second opening (25) of the second member (20), wherein the pusher element is configured to push the membrane (30) covering the second opening (25) in the unassembled state of the sample holder assembly (1) toward the first opening (24) of the second member (20), such that the membrane (30) adopts a pre-shaped form, such that when inserting the wall portion (12) of the first member (10) in the second volume (V2), membrane deformation forces are reduced due to the pre-shaped form of the membrane (30).

9. The sample holder assembly (1) according to one of the preceding claims, wherein the sample holder assembly (1) comprises a plurality of the first members (10, 10') that are integrally formed with each other and a plurality of the second members (20, 20') that are integrally formed with each other and wherein the first and second members (10, 10', 20, 20') have spatially corresponding wall portions (12, 22) such that an array of corresponding first and second members (10, 10', 20, 20') is formed.

10. The sample holder assembly (1) according to claims 8 and 9, wherein the pusher element has a plurality of integrally formed bodies arranged correspondingly to the integrally formed second members (20, 20'), such that in the unassembled state of the sample holder assembly (1), the pusher element is configured to simultaneously push the membrane (30) in the second volumes (V2) formed by the wall portions (22) of the second members (20, 20'), such that the membrane (30) adopts a pre-shaped form, such that when inserting the wall portions (12) of the first members (10, 10') in the second volumes (V2), membrane deformation forces are reduced due to the pre-shaped form of the membrane (30).

11. The sample holder assembly (1) according to any of the preceding claims, wherein the membrane (30) is flexible and plastically deformable, such the membrane (30) maintains a shape, particularly a shape of the non-even portion (31).

12. An optical microscope comprising a sample holder assembly (1) according to any of the preceding claims, wherein the microscope comprises at least one of the following imaging modalities:
- digital light sheet microscopy,
- wide-field microscopy,
- confocal microscopy, and/or
- optical microscopy.

13. Method for imaging a sample with the sample holder assembly according to any of the claims 1 to 11 or the microscope according to claim 12, the method comprising at least the steps of:
- Arranging the membrane (30) with a second side (30-2) of the membrane (30) that is opposite the first side (30-1) of the membrane (30) on the second opening (25) of the second member (20) or the second openings of the second members (20, 20'),
- Inserting the wall portion (12) of the first member (10) into the second volume (V2) such that the membrane (30) covers the first opening (14) and the liquid-tight seal is formed between the wall portions (12, 22) of the first and the second member (10, 20), thereby creating a sample imaging volume (VS) in the first volume (V1),
- Removing a first and second movable shape support member (41, 42) from the sample holder assembly (1),
- Growing the sample (100) on the first side (30-1) of the membrane (30) or arranging the sample (100) on the first side (30-1) of the membrane (30) in the first volume (V1),
- Imaging the sample.

## Patentansprüche

1. Eine Probenhalteranordnung (1) für die Mikroskopie, die mindestens die folgenden Komponenten umfasst:
- Ein erstes Element (10) mit einer ersten Öffnung (14) auf einer ersten Seite des ersten Elements (10) und einem Umfangswandabschnitt (12), der seitlich ein erstes Volumen (V1) umschließt, das die erste Öffnung (14) umfasst, wobei der Umfangswandabschnitt (12) des ersten Elements (10) eine nach innen gerichtete Seite (12-1) hat, die dem ersten Volumen (V1) zugewandt ist, und eine nach außen gerichtete Seite (12-2) gegenüber der nach innen gerichteten Seite (12-1),
- Ein zweites Element (20) mit einer ersten Öffnung (24) an einer ersten Seite des zweiten Elements (20) und einen Umfangswandabschnitt (22), der seitlich ein zweites Volumen (V2) umschließt, wobei der Umfangswandabschnitt (22) des zweiten Elements (20) eine nach innen gerichtete Seite (22-1) aufweist, die dem zweiten Volumen (V2) zugewandt ist,
wobei eine Form der nach außen gerichteten Seite (12-2) des Wandabschnitts (12) des ersten Elements (10) komplementär zu der nach innen gerichteten Seite (22-1) des Wandabschnitts (22) des zweiten Elements (20) ist, so dass der Wandabschnitt (12) des ersten Elements (10) in das zweite Volumen (V2) eingesetzt werden kann, so dass das erste Volumen (V1) zumindest teilweise von dem zweiten Volumen (V2) umfasst wird.
- Eine transparente Membran (30) mit einer ersten Seite (30-1) zum Halten einer Probe (100), wobei die Probenhalteranordnung (1) so konfiguriert ist, dass sie einen zusammengebauten Zustand einnehmen kann, wobei sich die Membran (30) im zusammengebauten Zustand zwischen der nach außen gerichteten Seite (12-2) des Wandabschnitts (12) des ersten Elements (10) der nach innen gerichteten Seite (22-1) des Wandabschnitts (22) des zweiten Elements (20) erstreckt und die erste Öffnung (14) des ersten Elements (10) abdeckt, wodurch eine flüssigkeitsdichte Abdichtung zwischen den Wandabschnitten (12, 22) des ersten und des zweiten Elements (10, 20) und ein transparenter Bildgebungsabschnitt für die Mikroskopie an der ersten Öffnung (14) des ersten Elements (10) gebildet wird,
**dadurch gekennzeichnet, dass**
der Wandabschnitt (22) des zweiten Elements (20) mindestens zwei membranformende Abschnitte (23-1, 23-2) umfasst, die aus einer Ebene, die die erste Öffnung (24) des zweiten Elements (20) umfasst, hervorstehen oder zurücktreten, wobei der Wandabschnitt (12) des ersten Elements (10) mindestens zwei membranformende Abschnitte (13-1, 13-2) aufweist, die komplementär zu den membranformenden Abschnitten (23-1, 23-2) des zweiten Elements (20) ausgebildet sind, wobei im zusammengebauten Zustand der Probenhalteranordnung (1) die Membran (30) zwischen den komplementär ausgebildeten membranformenden Abschnitten (13-1, 13-2, 23-1, 23-2) angeordnet ist, so dass die Membran (30) einen unebenen Abschnitt (31) zwischen den beiden membranformenden Abschnitten (13-1, 13-2, 23-1, 23-2) bildet, wobei der unebene Abschnitt (31) aus einer Ebene, die die erste Öffnung (14) des ersten Elements (10) umfasst, hervorsteht oder zurücktritt.

2. Die Probenhalteranordnung (1) gemäß Anspruch 1, wobei im zusammengebauten Zustand die Membran (30) zwischen den Wandabschnitten (12, 22) des ersten und des zweiten Elements (10, 20) reibschlüssig ist, insbesondere wobei die Membran (30) in der Probenhalteranordnung (1) nur durch die Reibungsverbindung zwischen der Membran (30) und den Wandabschnitten (12, 22) des ersten und des zweiten Elements (10, 20) fixiert ist.

3. Die Probenhalteranordnung (1) gemäß einem der Ansprüche 1 oder 2, wobei das zweite Element (20) eine zweite Öffnung (25) gegenüber der ersten Öffnung (24) aufweist, wobei die zweite Öffnung (25) durch den Wandabschnitt (22) des zweiten Elements (20) verbunden ist, wobei die Probenhalteranordnung (1) so konfiguriert ist, dass sie einen nicht zusammengebauten Zustand einnehmen kann, in dem die Membran (30) die zweite Öffnung (25) abdeckt, und wobei die Probenhalteranordnung (1) so konfiguriert ist, dass sie durch Einführen des Wandabschnitts (12) des ersten Elements (10) in die zweite Öffnung (25) und durch Bewegen des ersten Elements (10) in Richtung der ersten Öffnung (24) des zweiten Elements (20) vom zusammengebauten Zustand in den nicht zusammengebauten Zustand übergeht, wodurch die Membran (30) von der zweiten Öffnung (25) zur ersten Öffnung (24) des zweiten Elements (20) bewegt wird, wodurch die flüssigkeitsdichte Abdichtung zwischen den Wandabschnitten (12, 22) des ersten und des zweiten Elements (10, 20) gebildet wird, wobei insbesondere die Probenhalteranordnung (1) so konfiguriert ist, dass sie wiederholt vom zusammengebauten Zustand in den nicht zusammengebauten Zustand und umgekehrt übergeht.

4. Die Probenhalteranordnung (1) gemäß einem der vorstehenden Ansprüche, wobei die membranformenden Abschnitte (23-1, 23-2) des zweiten Elements (20) einen sägezahnförmigen Querschnitt aufweisen, so dass die Membran (30) an dem unebenen Abschnitt (31) in Richtung des zweiten Volumens (V2) in einem Keilwinkel vorsteht, der durch die membranformenden Abschnitte (23-1, 23-2) des zweiten Elements (20) erzwungen wird.

5. Die Probenhalteranordnung (1) gemäß einem der vorstehenden Ansprüche, wobei die Membranformungsabschnitte (23-1, 23-2) des zweiten Elements (20) einen konvexen Querschnitt, einen konkaven Querschnitt, einen V-förmigen Querschnitt, einen U-förmigen Querschnitt und/oder einen rechteckigen Querschnitt aufweisen, so dass im zusammengebauten Zustand der Probenhalteranordnung (1) der unebene Abschnitt (31) der Membran (30) die entsprechende Form der membranformenden Abschnitte (23-1, 23-2) annimmt.

6. Die Probenhalteranordnung (1) gemäß einem der vorstehenden Ansprüche, wobei die Probenhalteranordnung (1) ferner umfasst:
- ein erstes bewegliches Formstützelement (41), das zwischen die mindestens zwei membranformenden Abschnitte (13-1, 13-2) des ersten Elements (10) passt, wobei das erste Formstützelement (41) eine membranseitige Seite aufweist, die die gleiche Querschnittsform wie der membranformende Abschnitt (13-1, 13-2) des ersten Elements (10)
- ein zweites bewegliches Formstützelement (42), das zwischen die mindestens zwei membranformenden Abschnitte (23-1, 23-2) des zweiten Elements (20) passt, wobei das zweite Membranformstützelement (42) eine membranseitige Seite aufweist, die die gleiche Querschnittsform wie der membranformende Abschnitt (23-1, 23-2) des zweiten Elements (20) aufweist, wobei das erste und das zweite Membranstützelement (41, 42), wenn sie zwischen den membranformenden Abschnitten (13-1, 13-2,23-1, 23-2) angeordnet sind, sich entlang des unebenen Abschnitts (31) der Membran (30) von gegenüberliegenden Seiten (30-1, 30-2) der Membran (30) mit ihren entsprechenden membranseitigen Seiten zur Membran (30) hin ausgerichtet erstrecken, so dass die Membran (30) zwischen dem ersten und dem zweiten Membranformstützelement (41, 42) angeordnet ist, so dass eine Membrankontur entlang des unebenen Abschnitts (31) der Membran (30) entsprechend der Querschnittsform der membranformenden Abschnitte (13-1, 13-2, 23-1, 23-2) des ersten und des zweiten Elements (10, 20) über der ersten Öffnung (14) des ersten Elements (10) gebildet wird.

7. Die Probenhalteranordnung (1) gemäß einem der vorstehenden Ansprüche, wobei der unebene Membranabschnitt (31) eine Höhe über der Ebene aufweist, die die erste Öffnung (14) umfasst, wobei die Höhe mindestens 1 mm beträgt, so dass der unebene Membranabschnitt (31) das Probenvolumen (VS) in eine erste und eine zweite Kammer trennt, wobei die Kammern fluidisch voneinander getrennt sind.

8. Die Probenhalteranordnung (1) gemäß einem der vorstehenden Ansprüche, wobei die Probenhalteranordnung (1) ein Schubelement mit einem Körper umfasst, wobei der Körper so geformt ist, dass der Körper zumindest in einer Ausrichtung des Körpers in ein zweites Volumen (V2) passt, so dass der Körper von der Seite der zweiten Öffnung (25) des zweiten Elements (20) in das zweite Volumen (V2) eingeführt werden kann, wobei das Schubelement so konfiguriert ist, dass es die Membran (30), die die zweite Öffnung (25) im nicht zusammengebauten Zustand der Probenhalteranordnung (1) abdeckt, in Richtung der ersten Öffnung (24) des zweiten Elements (20) zu drücken, so dass die Membran (30) eine vorgeformte Form annimmt, so dass beim Einführen des Wandabschnitts (12) des ersten Elements (10) in das zweite Volumen (V2) Membranverformungskräfte aufgrund der vorgeformten Form der Membran (30) reduziert werden.

9. Die Probenhalteranordnung (1) gemäß einem der vorstehenden Ansprüche, wobei die Probenhalteranordnung (1) eine Vielzahl der ersten Elemente (10, 10'), die integral miteinander ausgebildet sind, und eine Vielzahl der zweiten Elemente (20, 20'), die integral miteinander ausgebildet sind, umfasst, und wobei die ersten und zweiten Elemente (10, 10', 20, 20') räumlich korrespondierende Wandabschnitte (12, 22) aufweisen, so dass eine Anordnung korrespondierender erster und zweiter Elemente (10, 10', 20, 20') gebildet ist.

10. Die Probenhalteranordnung (1) gemäß den Ansprüchen 8 und 9, wobei das Schubelement mehrere integral miteinander ausgebildete Körper aufweist, die entsprechend den integral miteinander ausgebildeten zweiten Elementen (20, 20') angeordnet sind, so dass im nicht zusammengebauten Zustand der Probenhalteranordnung (1) das Schubelement so ausgebildet ist, dass es gleichzeitig die Membran (30) in den durch die Wandabschnitte (22) der zweiten Elemente (20, 20') gebildeten zweiten Volumen (V2) schiebt, so dass die Membran (30) eine vorgeformte Form annimmt, so dass beim Einführen der Wandabschnitte (12) der ersten Elemente (10, 10') in die zweiten Volumen (V2) Membranverformungskräfte aufgrund der vorgeformten Form der Membran (30) reduziert werden.

11. Die Probenhalteranordnung (1) gemäß einem der vorstehenden Ansprüche, wobei die Membran (30) flexibel und plastisch verformbar ist, so dass die Membran (30) eine Form, insbesondere eine Form des unebenen Abschnitts (31), beibehält.

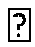 12. ptisches Mikroskop mit einer Probenhalteranordnung (1) gemäß einem der vorstehenden Ansprüche, wobei das Mikroskop mindestens eine der folgenden Bildgebungsmodalitäten umfasst:
- digitale Lichtblattmikroskopie,
- Weitfeldmikroskopie,
- konfokale Mikroskopie und/oder
- optische Mikroskopie.

13. Verfahren zum Abbilden einer Probe mit der Probenhalterungsanordnung gemäß einem der Ansprüche 1 bis 11 oder dem Mikroskop gemäß Anspruch 12, wobei das Verfahren mindestens die folgenden Schritte umfasst:
- Anordnen der Membran (30) mit einer zweiten Seite (30-2) der Membran (30), die der ersten Seite (30-1) der Membran (30) gegenüberliegt, an der zweiten Öffnung (25) des zweiten Elements (20) oder den zweiten Öffnungen der zweiten Elemente (20, 20'),
- Einsetzen des Wandabschnitts (12) des ersten Elements (10) in das zweite Volumen (V2), so dass die Membran (30) die erste Öffnung (14) abdeckt und die flüssigkeitsdichte Abdichtung zwischen den Wandabschnitten (12, 22) des ersten und des zweiten Elements (10, 20) gebildet wird, wodurch ein Probenbildgebungsvolumen (VS) im ersten Volumen (V1) gebildet wird,
- Entfernen eines ersten und eines zweiten beweglichen Formstützelements (41, 42) aus der Probenhalteranordnung (1),
- Wachsen der Probe (100) auf der ersten Seite (30-1) der Membran (30) oder Anordnen der Probe (100) auf der ersten Seite (30-1) der Membran (30) im ersten Volumen (V1)
- Abbilden der Probe.

## Revendications

1. Ensemble de porte-échantillons (1) pour microscopie comprenant au moins les composants suivants :
- un premier élément (10) ayant une première ouverture (14) sur un premier côté du premier élément (10), et une portion de paroi circonférentielle (12) renfermant latéralement un premier volume (V1) comprenant la première ouverture (14), dans lequel la portion de paroi circonférentielle (12) du premier élément (10) a un côté tourné vers l'intérieur (12-1) qui est tourné vers le premier volume (V1) et un côté tourné vers l'extérieur (12-2) à l'opposé du côté tourné vers l'intérieur (12-1),
- un second élément (20) ayant une première ouverture (24) sur un premier côté du second élément (20), et une portion de paroi circonférentielle (22) renfermant latéralement un second volume (V2), dans lequel la portion de paroi circonférentielle (22) du second élément (20) a un côté tourné vers l'intérieur (22-1) tourné vers le second volume (V2),
dans lequel une forme du côté tourné vers l'extérieur (12-2) de la portion de paroi (12) du premier élément (10) est complémentaire au côté tourné vers l'intérieur (22-1) de la portion de paroi (22) du second élément (20) de sorte que la portion de paroi (12) du premier élément (10) peut être insérée dans le second volume (V2), de sorte que le premier volume (V1) est au moins partiellement compris par le second volume (V2),
- une membrane transparente (30) ayant un premier côté (30-1) pour supporter un échantillon (100), dans lequel l'ensemble de porte-échantillons (1) est configuré pour adopter un état assemblé, dans lequel dans l'état assemblé, la membrane (30) s'étend entre le côté tourné vers l'extérieur (12-2) de la portion de paroi (12) du premier élément (10), le côté tourné vers l'intérieur (22-1) de la portion de paroi (22) du second élément (20), et recouvre la première ouverture (14) du premier élément (10), formant de ce fait un joint étanche au liquide entre les portions de paroi (12, 22) du premier et du second élément (10, 20) et une portion d'imagerie transparente pour microscopie au niveau de la première ouverture (14) du premier élément (10),
**caractérisé en ce que**
la portion de paroi (22) du second élément (20) comprend au moins deux portions de mise en forme de membrane (23-1, 23-2) qui font saillie ou reculent depuis un plan comprenant la première ouverture (24) du second élément (20), dans lequel la portion de paroi (12) du premier élément (10) comprend au moins deux portions de mise en forme de membrane (13-1, 13-2) formées de manière complémentaire aux portions de mise en forme de membrane (23-1, 23-2) du second élément (20), dans lequel dans l'état assemblé de l'ensemble de porte-échantillons (1), la membrane (30) est agencée entre les portions de mise en forme de membrane formées complémentaires (13-1, 13-2, 23-1, 23-2) de sorte que la membrane (30) forme une portion non régulière (31) entre les deux portions de mise en forme de membrane (13-1, 13-2, 23-1, 23-2), dans lequel la portion non régulière (31) fait saillie ou recule depuis un plan comprenant la première ouverture (14) du premier élément (10).

2. Ensemble de porte-échantillons (1) selon la revendication 1, dans lequel dans l'état assemblé, la membrane (30) est montée par friction entre les portions de paroi (12, 22) du premier et du second élément (10, 20), notamment dans lequel la membrane (30) est fixée dans l'ensemble de porte-échantillons (1) uniquement au moyen de la connexion à friction entre la membrane (30) et les portions de paroi (12, 22) du premier et du second élément (10, 20).

3. Ensemble de porte-échantillons (1) selon l'une quelconque des revendications 1 ou 2, dans lequel le second élément (20) comprend une seconde ouverture (25) à l'opposé de la première ouverture (24), la seconde ouverture (25) étant connectée par la portion de paroi (22) du second élément (20), dans lequel l'ensemble de porte-échantillons (1) est configuré pour adopter un état démonté dans lequel la membrane (30) recouvre la seconde ouverture (25), et dans lequel l'ensemble de porte-échantillons (1) est configuré pour passer de l'état démonté à l'état assemblé en insérant la portion de paroi (12) du premier élément (10) dans la seconde ouverture (25) et déplaçant le premier élément (10) vers la première ouverture (24) du second élément (20), déplaçant de ce fait la membrane (30) de la seconde ouverture (25) à la première ouverture (24) du second élément (20), formant de ce fait le joint étanche au liquide entre les portions de paroi (12, 22) du premier et du second élément (10, 20), notamment dans lequel l'ensemble de porte-échantillons (1) est configuré pour passer de manière répétée de l'état assemblé à l'état démonté et vice versa.

4. Ensemble de porte-échantillons (1) selon une des revendications précédentes, dans lequel les portions de mise en forme de membrane (23-1, 23-2) du second élément (20) ont une section transversale en forme de dent de scie de sorte que la membrane (30) fait saillie au niveau de la portion non régulière (31) vers le second volume (V2) à un angle de bec imposé par les portions de mise en forme de membrane (23-1, 23-2) du second élément (20).

5. Ensemble de porte-échantillons (1) selon une des revendications précédentes, dans lequel les portions de mise en forme de membrane (23-1, 23-2) du second élément (20) ont une section transversale de forme convexe, une section transversale de forme concave, une section transversale en forme de V, une section transversale en forme de U et/ou une section transversale de forme rectangulaire de sorte que dans l'état assemblé de l'ensemble de porte-échantillons (1), la portion non régulière (31) de la membrane (30) adopte la forme correspondante des portions de mise en forme de membrane (23-1, 23-2).

6. Ensemble de porte-échantillons (1) selon une des revendications précédentes, dans lequel l'ensemble de porte-échantillons (1) comprend en outre :
- un premier élément de support de forme mobile (41) qui s'ajuste entre les au moins deux portions de mise en forme de membrane (13-1, 13-2) du premier élément (10), dans lequel le premier élément de support de forme (41) a un côté tourné vers la membrane qui a la même forme en coupe transversale que la portion de mise en forme de membrane (13-1, 13-2) du premier élément (10),
- un second élément de support de forme mobile (42) qui s'ajuste entre les au moins deux portions de mise en forme de membrane (23-1, 23-2) du second élément (20), dans lequel le second élément de support de forme de membrane (42) a un côté tourné vers la membrane qui a la même forme en coupe transversale que la portion de mise en forme de membrane (23-1, 23-2) du second élément (20), dans lequel le premier et le second éléments de support de membrane (41, 42) s'étendent le long de la portion non régulière (31) de la membrane (30) depuis des côtés opposés (30-1, 30-2) de la membrane (30) avec les côtés tournés vers la membrane correspondants orientés vers la membrane (30) lorsqu'ils sont agencés entre les portions de mise en forme de membrane (13-1, 13-2, 23-1, 23-2) de sorte que la membrane (30) est agencée entre les premier et second éléments de support de forme de membrane (41, 42) de sorte qu'un contour de membrane le long de la portion non régulière (31) de la membrane (30) est formé pour correspondre à la forme en coupe transversale des portions de mise en forme de membrane (13-1, 13-2, 23-1, 23-2) du premier et du second élément (10, 20) en travers de la première ouverture (14) du premier élément (10).

7. Ensemble de porte-échantillons (1) selon une des revendications précédentes, dans lequel la portion de membrane non régulière (31) a une hauteur au-dessus du plan comprenant la première ouverture (14), dans lequel ladite hauteur est d'au moins 1 mm de sorte que la portion de membrane non régulière (31) sépare le volume d'échantillon (VS) dans un premier et un second compartiment, lesquels compartiments sont déconnectés de manière fluidique.

8. Ensemble de porte-échantillons (1) selon l'une quelconque des revendications précédentes, dans lequel l'ensemble de porte-échantillons (1) comprend un élément de poussoir ayant un corps, dans lequel le corps est formé de sorte qu'au moins dans une orientation du corps, le corps s'ajuste dans le second volume (V2) de sorte que le corps peut être inséré dans le second volume (V2) depuis le côté de la seconde ouverture (25) du second élément (20), dans lequel l'élément de poussoir est configuré pour pousser la membrane (30) recouvrant la seconde ouverture (25) dans l'état démonté de l'ensemble de porte-échantillons (1) vers la première ouverture (24) du second élément (20) de sorte que la membrane (30) adopte une forme préformée de sorte que lors de l'insertion de la portion de paroi (12) du premier élément (10) dans le second volume (V2), des forces de déformation de membrane sont réduites en raison de la forme préformée de la membrane (30).

9. Ensemble de porte-échantillons (1) selon une des revendications précédentes, dans lequel l'ensemble de porte-échantillons (1) comprend une pluralité des premiers éléments (10, 10') qui sont intégralement formés l'un avec l'autre et une pluralité des seconds éléments (20, 20') qui sont intégralement formés l'un avec l'autre, et dans lequel les premiers et seconds éléments (10, 10', 20, 20') ont des portions de paroi à correspondance spatiale (12, 22) de sorte qu'un réseau de premiers et seconds éléments correspondants (10, 10', 20, 20') est formé.

10. Ensemble de porte-échantillons (1) selon les revendications 8 et 9, dans lequel l'élément de poussoir a une pluralité de corps formés intégralement agencés pour correspondre aux seconds éléments formés intégralement (20, 20') de sorte que dans l'état démonté de l'ensemble de porte-échantillons (1), l'élément de poussoir est configuré pour pousser simultanément la membrane (30) dans les seconds volumes (V2) formés par les portions de paroi (22) des seconds éléments (20, 20') de sorte que la membrane (30) adopte une forme préformée de sorte que lors de l'insertion des portions de paroi (12) des premiers éléments (10, 10') dans les seconds volumes (V2), des forces de déformation de membrane sont réduites en raison de la forme préformée de la membrane (30).

11. Ensemble de porte-échantillons (1) selon l'une quelconque des revendications précédentes, dans lequel la membrane (30) est flexible et déformable de manière plastique de sorte que la membrane (30) maintient une forme, notamment une forme de la portion non régulière (31).

12. Microscope optique comprenant un ensemble de porte-échantillons (1) selon l'une quelconque des revendications précédentes, dans lequel le microscope comprend au moins une des modalités d'imagerie suivantes :
- microscopie à nappe de lumière numérique,
- microscopie à champ large,
- microscopie confocale, et/ou
- microscopie optique.

13. Procédé d'imagerie d'un échantillon avec l'ensemble de porte-échantillons selon l'une quelconque des revendications 1 à 11 ou le microscope selon la revendication 12, le procédé comprenant au moins les étapes consistant à :
- agencer la membrane (30) avec un second côté (30-2) de la membrane (30) qui est opposé au premier côté (30-1) de la membrane (30) sur la seconde ouverture (25) du second élément (20) ou les secondes ouvertures des seconds éléments (20, 20'),
- insérer la portion de paroi (12) du premier élément (10) dans le second volume (V2) de sorte que la membrane (30) recouvre la première ouverture (14) et le joint étanche au liquide est formé entre les portions de paroi (12, 22) du premier et du second élément (10, 20), créant de ce fait un volume d'imagerie d'échantillon (VS) dans le premier volume (V1),
- retirer un premier et second élément de support de forme mobile (41, 42) de l'ensemble de porte-échantillons (1),
- faire pousser l'échantillon (10) sur le premier côté (30-1) de la membrane (30) ou agencer l'échantillon (10) sur le premier côté (30-1) de la membrane (30) dans le premier volume (V1),
- imager l'échantillon.
